# EUROPEAN PATENT APPLICATION

(11) **EP 4 008 401 A1**
(43) Date of publication of application: **08.06.2022**
(21) Application number: 20849263.7
(22) Date of filing: 21.07.2020
(51) Int. Cl.: A61P 19/04, A61P 43/00, A61K 31/352, A61K 31/353

(54) **COMPOSITION FOR CARTILAGE REGENERATION PROMOTION**

(30) Priority: 02.08.2019 JP 2019143144
(71) Applicant: Suntory Holdings Limited, Osaka 530-8203 (JP)
(72) Inventor: OTSUKA, Yuta, Soraku-gun, Kyoto 619-0284 (JP); FUNAKI, Ayuta, Soraku-gun, Kyoto 619-0284 (JP)
(74) Representative: Vossius & Partner Patentanwälte Rechtsanwälte mbB
(86) International application number: PCT/JP2020/028306
(87) International publication number: WO 2021/024801

(57) **Abstract**

The present invention aims to provide a composition for promoting cartilage regeneration, which promotes cartilage regeneration, and a method of promoting cartilage regeneration. The present invention relates to a composition for promoting cartilage regeneration, containing a flavonoid represented by the following formula (1) as an active ingredient: 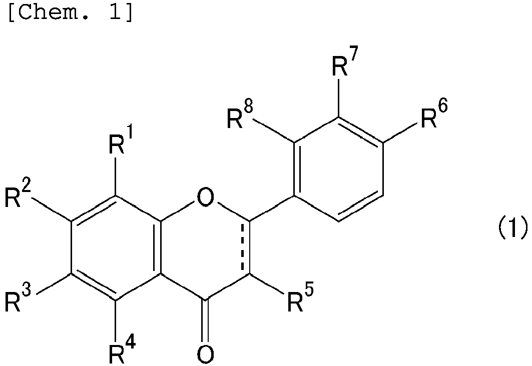 wherein R¹, R², R³, R⁴ and R⁵ each independently represent a hydrogen atom or a hydroxy group; R⁶ represents a hydroxy group or a C1-C2 alkoxy group; one of R⁷ and R⁸ represents a hydrogen atom and the other represents a hydroxy group or a C1-C2 alkoxy group; and a dashed line indicates that a bond may or may not be present.

## Description

### TECHNICAL FIELD

The present invention relates to a composition for promoting cartilage regeneration. The present invention also relates to a method of promoting cartilage regeneration, for example.

### BACKGROUND ART

Cartilage is a connective tissue consisting of cartilage cells and a matrix surrounding the cells. In animals such as human, cartilage makes up joints, skeleton, and the like. Articular cartilage thinly covers joint bone surfaces and has a function to keep the joint motion fluid. A decrease in cartilage due to aging or the like will impair joint functions.

As a composition capable of protecting cartilage, Patent Literature 1 discloses a composition containing glucosamine and at least one component selected from the group consisting of a plant material, an amino acid, a polyphenol, a vitamin, and a mineral, and the like as active ingredients.

### CITATION LIST

### - Patent Literature

Patent Literature 1: JP 2017-14164 A

### SUMMARY OF INVENTION

### - Technical Problem

The composition of Patent Literature 1 protects cartilage by suppressing destruction of articular cartilage cells. However, once cartilage cells are destroyed due to aging or the like, the cartilage that has been reduced cannot be regenerated by suppressing destruction of articular cartilage cells. Patent Literature 1 nowhere examines a substance effective in promoting cartilage regeneration.

The present invention aims to provide a composition for promoting cartilage regeneration, which promotes cartilage regeneration. The present invention also aims to provide a method of promoting cartilage regeneration.

### - Solution to Problem

As a result of extensive studies to solve the above issue, the present inventors found that a flavonoid having a specific structure has an action that promotes differentiation into cartilage cells and that such a flavonoid is useful in promoting cartilage regeneration. A technique that can promote differentiation into cartilage cells is a useful technique that can regenerate cartilage particularly even after the cartilage is destroyed and reduced. Cartilage can be grown and regenerated by promoting differentiation of mesenchymal stem cells into cartilage cells.

Specifically, the present invention relates to a composition for promoting cartilage regeneration, a method of promoting cartilage regeneration, and the like described below.
[1] A composition for promoting cartilage regeneration, containing a flavonoid represented by the following formula (1) as an active ingredient: 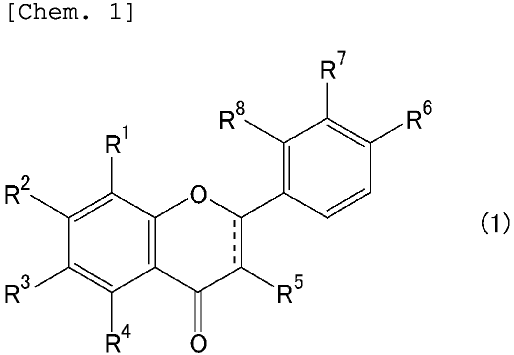 wherein R¹, R², R³, R⁴ and R⁵ each independently represent a hydrogen atom or a hydroxy group; R⁶ represents a hydroxy group or a C1-C2 alkoxy group; one of R⁷ and R⁸ represents a hydrogen atom and the other represents a hydroxy group or a C1-C2 alkoxy group; and a dashed line indicates that a bond may or may not be present.
[2] The composition for promoting cartilage regeneration according to [1] above, wherein the composition promotes differentiation into cartilage cells.
[3] The composition for promoting cartilage regeneration according to [1] or [2] above, wherein R¹ and R³ represent hydrogen atoms.
[4] The composition for promoting cartilage regeneration according to any one of [1] to [3] above, wherein R² and R⁴ represent hydroxy groups.
[5] The composition for promoting cartilage regeneration according to any one of [1] to [4] above, wherein the flavonoid represented by the formula (1) is a flavonoid represented by the following formula (2):

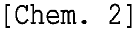

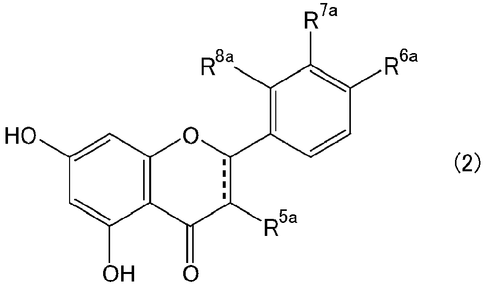

   wherein R^{5a} represents a hydrogen atom or a hydroxy group; R^{6a} represents a hydroxy group or a methoxy group; one of R^{7a} and R^{8a} represents a hydrogen atom and the other represents a hydroxy group or a methoxy group; and a dashed line indicates that a bond may or may not be present.
[6] The composition for promoting cartilage regeneration according to any one of [1] to [5] above, wherein the flavonoid represented by the formula (1) is at least one selected from the group consisting of quercetin, hesperetin, taxifolin, isorhamnetin, eriodictyol, luteolin, and morin.
[7] A method of promoting cartilage regeneration, including: administering a flavonoid represented by the above formula (1).
[8] Use of the flavonoid represented by the formula (1) for promoting cartilage regeneration.

### - Advantageous Effects of Invention

The present invention provides a composition for promoting cartilage regeneration, which promotes cartilage regeneration. The present invention also provides a method of promoting cartilage regeneration.

### DESCRIPTION OF EMBODIMENTS

The composition for promoting cartilage regeneration of the present invention contains a flavonoid represented by the following formula (1) as an active ingredient. In the present invention, preferably, the cartilage is an articular cartilage.

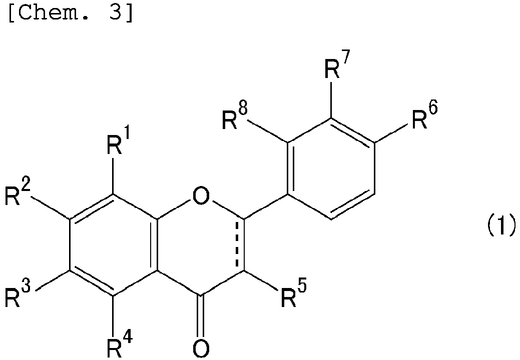

In the formula, R¹, R², R³, R⁴ and R⁵ each independently represent a hydrogen atom or a hydroxy group; R⁶ represents a hydroxy group or a C1-C2 alkoxy group; one of R⁷ and R⁸ represents a hydrogen atom and the other represents a hydroxy group or a C1-C2 alkoxy group; and a dashed line indicates that a bond may or may not be present.

The flavonoid represented by the formula (1) may be of one type or two or more types. Herein, the flavonoid represented by the formula (1) is also simply referred to as "the flavonoid".

In the formula (1), preferably, R¹ and R³ are hydrogen atoms. Preferably, R² and R⁴ are hydroxy groups. Preferably, R⁵ is a hydrogen atom or a hydroxy group.

In the formula (1), the C1-C2 alkoxy group is a methoxy group or an ethoxy group, preferably a methoxy group.

Preferably, R⁶ is a hydroxy group or a methoxy group. When R⁷ represents a hydrogen atom, R⁸ represents a hydroxy group or a C1-C2 alkoxy group. When R⁸ represents a hydrogen atom, R⁷ represents a hydroxy group or a C1-C2 alkoxy group. Preferably, R⁷ is a hydrogen atom, a hydroxy group, or a methoxy group. Preferably, R⁸ is a hydrogen atom or a hydroxy group.

Preferably, the flavonoid represented by the formula (1) is a flavonoid represented by the following formula (2). The flavonoid represented by the formula (2) is a compound represented by the formula (1) wherein R¹ and R³ are hydrogen atoms; R² and R⁴ are hydroxy groups; R⁶ is a hydroxy group or a methoxy group; and one of R⁷ and R⁸ represents a hydrogen atom and the other represents a hydroxy group or a methoxy group. Use of the flavonoid represented by the following formula (2) can provide an excellent cartilage regeneration promoting effect.

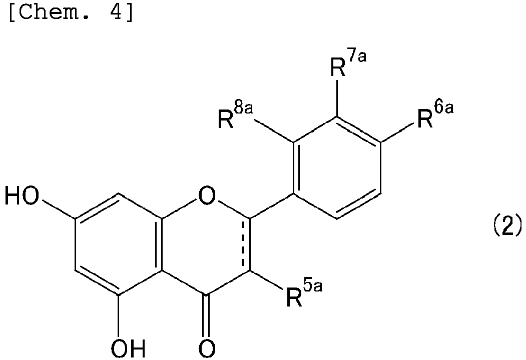

In the formula, R^{5a} represents a hydrogen atom or a hydroxy group; R^{6a} represents a hydroxy group or a methoxy group; one of R^{7a} and R^{8a} represents a hydrogen atom and the other represents a hydroxy group or a methoxy group; and a dashed line indicates that a bond may or may not be present.

In the formula (2), when R^{7a} represents a hydrogen atom, R^{8a} represents a hydroxy group or a methoxy group. When R^{8a} represents a hydrogen atom, R^{7a} represents a hydroxy group or a methoxy group. In one embodiment, preferably, R^{7a} is a hydrogen atom, a hydroxy group, or a methoxy group, and R^{8a} is a hydrogen atom or a hydroxy group.

Preferred flavonoids represented by the formula (1) are hesperetin, quercetin, taxifolin, isorhamnetin, eriodictyol, luteolin, and morin. These flavonoids each have a high cartilage regeneration promoting effect. These flavonoids are represented by the formula (2). Preferably, the composition for promoting cartilage regeneration of the present invention contains one or more of these flavonoids as active ingredients. More preferred flavonoids represented by the formula (1) are hesperetin, quercetin, taxifolin, isorhamnetin, eriodictyol, and morin.

The flavonoid represented by the formula (1) may have any origin. Since the flavonoid represented by the formula (1) is present in plants, it can be prepared by extraction from plants. Chemically synthesized flavonoids can also be used. In the present invention, the composition may contain, for example, a plant-derived material such as a plant extract containing the flavonoid represented by the formula (1), as long as the effect of the present invention is obtained.

The flavonoid represented by the formula (1) is a compound that is contained in natural products, food, and/or beverages and that has been used as a food ingredient. Thus, daily intake of the flavonoid is less likely to cause problems in terms of safety, for example. Thus, the present invention can provide a composition for promoting cartilage regeneration, which contains a highly safe substance as an active ingredient.

As shown in examples described later, culturing synovial cells in the presence of the flavonoid represented by the formula (1) increased the weight of the resultant cartilage pellets. The synovial cells used in the examples included mesenchymal stem cells, and the mesenchymal stem cells differentiated into cartilage cells, forming cartilage pellets. An increase in the weight of the cartilage pellets indicates that differentiation of mesenchymal stem cells into cartilage cells was promoted. Thus, the flavonoid represented by the formula (1) promotes differentiation of mesenchymal stem cells into cartilage cells. For example, it is said that mesenchymal stem cells present in the synovium and articular cartilage surface layer easily differentiate into cartilage cells. The flavonoid represented by the formula (1) is useful, for example, in promoting differentiation of synovial mesenchymal stem cells or mesenchymal stem cells present in the articular cartilage surface layer into cartilage cells.

Cartilage regeneration can be promoted by promoting differentiation into cartilage cells. Thus, the flavonoid represented by the formula (1) is useful in promoting cartilage regeneration. The composition for promoting cartilage regeneration of the present invention can be used to promote cartilage regeneration by promoting differentiation into cartilage cells. It is possible to increase cartilage by promoting cartilage regeneration. Thus, promoting cartilage regeneration is useful, for example, in preventing a reduction in cartilage. It is also possible to repair cartilage that has been reduced by promoting cartilage regeneration. The composition for promoting cartilage regeneration of the present invention can also be used as a composition for promoting differentiation of mesenchymal stem cells into cartilage cells.

The composition for promoting cartilage regeneration of the present invention is applicable for either therapeutic use (medical use) or non-therapeutic use (nonmedical use). The "non-therapeutic" is a concept that does not include medical activities, i.e., a concept that does not include surgery, therapy, or diagnosis of humans.

The composition for promoting cartilage regeneration of the present invention can be provided in the form of a food or beverage, a pharmaceutical product, a quasi-pharmaceutical product, feed, or the like. The composition for promoting cartilage regeneration of the present invention may be a food or beverage, a pharmaceutical product, a quasi-pharmaceutical product, feed, or the like by itself for promoting cartilage regeneration; or may be a material, a preparation, or the like to be added to such a product or the like.

For example, the composition for promoting cartilage regeneration of the present invention can be provided as an agent or the like, but it is not limited thereto. The agent can be provided directly as a composition, or can be provided as a composition containing the agent. In one embodiment, the composition for promoting cartilage regeneration of the present invention can also be referred to as a "cartilage regeneration promoter".

The composition for promoting cartilage regeneration of the present invention may be either an oral composition or a parenteral composition, preferably an oral composition. The oral composition may be a food or beverage, an oral pharmaceutical product, an oral quasi-pharmaceutical product, or feed, preferably a food or beverage or an oral pharmaceutical product, still more preferably a food or beverage.

The composition for promoting cartilage regeneration of the present invention can also contain any additives and/or any components in addition to the flavonoid, as long as the effect of the present invention is not impaired. Such additives and components can be selected according to the composition form or the like. Those that can be used generally in food, beverages, pharmaceutical products, quasi-pharmaceutical products, feed, and the like can be used. When the composition for promoting cartilage regeneration is provided as a food or beverage, a pharmaceutical product, a quasi-pharmaceutical product, feed, or the like, any common method can be used for the production.

For example, when the composition for promoting cartilage regeneration of the present invention is provided as a food or beverage, various types of food or beverages can be provided by adding a component usable in food or beverages (e.g., a food material or an optional food additive) to the flavonoid. Non-limiting examples of the food or beverage include general foods and beverages, health foods, health beverages, foods with function claims, foods for specified health uses, and foods and beverages for the sick. The health foods, the foods with function claims, the foods for specified health uses, and the like can be provided in various preparation forms, such as fine granules, tablets, granules, powders, capsules, chewable tablets, syrups, liquids, and liquid foods.

When the composition for promoting cartilage regeneration of the present invention is provided as a pharmaceutical product or quasi-pharmaceutical product, for example, various dosage forms of pharmaceutical products or quasi-pharmaceutical products can be provided by, for example, adding a pharmacologically acceptable carrier, an optional additive, or the like to the flavonoid. Such a carrier, an additive, or the like may be of any pharmacologically acceptable type that can be used in pharmaceutical products or quasi-pharmaceutical products. Examples include excipients, binders, disintegrants, lubricants, antioxidants, and colorants. One or more of these can be used. Examples of the form of administration (intake) of the pharmaceutical product or quasi-pharmaceutical product include oral administration and parenteral administration (e.g., transdermal, transmucosal, enteral administration, and injection). When the composition for promoting cartilage regeneration of the present invention is provided as a pharmaceutical product or quasi-pharmaceutical product, preferably, it is an oral pharmaceutical product or an oral quasi-pharmaceutical product. Examples of dosage forms for oral administration include liquids, tablets, powders, fine granules, granules, sugar-coated tablets, capsules, suspensions, emulsions, and chewable tablets. Examples of dosage forms for parenteral administration include injections, intravenous agents, and adhesive skin patches. The pharmaceutical product may be for non-human animals.

When the composition for promoting cartilage regeneration of the present invention is provided as feed, the flavonoid may be added to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of the flavonoid represented by the formula (1) in the composition for promoting cartilage regeneration of the present invention is not limited, and it can be set according to the form of the composition or the like.

For example, the amount of the flavonoid represented by the formula (1) in the composition for promoting cartilage regeneration of the present invention is preferably 0.0001 wt% or more, more preferably 0.001 wt% or more, and is preferably 90 wt% or less, more preferably 50 wt% or less in the composition. In one embodiment, the amount of the flavonoid represented by the formula (1) is preferably 0.0001 to 90 wt%, more preferably 0.001 to 50 wt% in the composition for promoting cartilage regeneration. The amount is the total amount when the composition for promoting cartilage regeneration contains two or more of the flavonoids.

The composition for promoting cartilage regeneration of the present invention may be given by any administration route, and can be fed or administered by a method suitable for the form. In one embodiment, preferably, the composition for promoting cartilage regeneration is orally fed (oral administration). The dosage (intake) of the composition for promoting cartilage regeneration of the present invention is not limited, as long as it is the amount that provides the effect of promoting differentiation into cartilage cells and the cartilage regeneration promoting effect. The dosage may be suitably set according to the dosage form, administration method, and the like.

In one embodiment, when the composition for promoting cartilage regeneration is fed or administered to a human (adult), the dosage of the flavonoid represented by the formula (1) is preferably 0.1 mg or more, more preferably 1 mg or more, and preferably 8000 mg or less, more preferably 4000 mg or less per 60 kg body weight per day. In one embodiment, the dosage of the flavonoid to a human (adult) is preferably 0.1 to 8000 mg, more preferably 1 to 4000 mg per 60 kg body weight per day. Preferably, the above amount of the flavonoid is fed or administered in one or more portions per day, for example, one to several portions (e.g., two to three portions) per day. In one embodiment, preferably, the above amount of the flavonoid is orally fed or administered to a human. In one embodiment of the present invention, the composition for promoting cartilage regeneration can be used to feed or administer the above amount of the flavonoid per 60 kg body weight per day to a human. The dosage is the total amount when the composition for promoting cartilage regeneration contains two or more of the flavonoids.

Preferably, the composition for promoting cartilage regeneration of the present invention is continuously fed or administered. Continuous feeding or administration of the flavonoid is likely to provide a higher cartilage regeneration promoting effect.

The composition for promoting cartilage regeneration of the present invention is useful in preventing or ameliorating a symptom or disease that is likely to be prevented or ameliorated by promoting cartilage regeneration. Examples of such a symptom or disease include those that develop due to a reduction in cartilage. Examples include cartilage defects, cartilage damage, and osteoarthritis (e.g., knee osteoarthritis).

Herein, prevention of a symptom or disease includes preventing the onset of a symptom or disease, delaying the onset of a symptom or disease, reducing the incidence of a symptom or disease, reducing the onset risk of a symptom or disease, and the like. Amelioration of a symptom or disease includes helping a subject recover from a symptom or disease, alleviating a symptom or disease, delaying or preventing symptom exacerbation or disease progression, and the like.

A subject (or "subject for administration") to be fed or administered with the composition for promoting cartilage regeneration of the present invention is not limited. The subject is preferably a human or non-human mammal, more preferably a human.

In one embodiment, examples of the subject for administration include one who needs or wants to promote cartilage regeneration. The composition for promoting cartilage regeneration of the present invention can also be used for a healthy person, for example, for purposes such as prevention of a symptom or disease that is likely to be prevented or ameliorated by promoting cartilage regeneration.

The composition for promoting cartilage regeneration of the present invention may be labeled with a function claim based on promotion of cartilage regeneration. The composition for promoting cartilage regeneration of the present invention may be labeled with one or more function claims, such as "maintaining cartilage mass", "preventing or reducing cartilage wear", and "preventing or reducing cartilage degeneration".

In one embodiment of the present invention, preferably, the composition for promoting cartilage regeneration of the present invention is a food or beverage labeled with one or more function claims described above. The labels indicating the above function claims may be labels indicating use for providing these functions.

In one embodiment, the composition for promoting cartilage regeneration of the present invention can be used as a reagent for culturing cartilage cells, for example. The composition for promoting cartilage regeneration of the present invention may be a composition for promoting regeneration of cultured cartilage. Culturing mesenchymal stem cells in a medium containing the flavonoid represented by the formula (1) can promote differentiation of the cells into cartilage cells. When the composition for promoting cartilage regeneration is used to culture cartilage cells, preferably, the composition is used in an amount such that the amount of the flavonoid represented by the formula (1) in the medium is preferably 0.1 to 100 µM, more preferably 1 to 50 µM.

The present invention also encompasses the following method and use:
a method of promoting cartilage regeneration, including administering the flavonoid represented by the formula (1); and
use of the flavonoid represented by the formula (1) for promoting cartilage regeneration.

The method and the use may be therapeutic or non-therapeutic. Administering (feeding) the flavonoid represented by the formula (1) to a subject can promote differentiation of mesenchymal stem cells into cartilage cells. Thus, cartilage regeneration can be promoted. Promoting cartilage regeneration is useful in, for example, preventing or ameliorating the symptoms or diseases described above, such as cartilage defects, cartilage damage, and osteoarthritis (e.g., knee osteoarthritis).

In the above method and use, the flavonoid and its preferred embodiment are as described above for the composition for promoting cartilage regeneration of the present invention. One of the flavonoids represented by the formula (1) may be used, or two or more thereof may be used. In the above method and use, preferably, the flavonoid represented by the formula (1) is fed (administered) at least once a day, for example, once to several times (for example, two or three times) a day. The above use is preferably for humans or non-human mammals, more preferably for humans.

In the above method and use, the flavonoid represented by the formula (1) may be used in an amount (effective amount) that provides the action that promotes cartilage regeneration. A preferred dosage of the flavonoid, a preferred subject for administration, and the like are as described above for the composition for promoting cartilage regeneration of the present invention. The flavonoid represented by the formula (1) may be administered directly or may be administered as a composition containing the flavonoid. For example, the composition for promoting cartilage regeneration of the present invention may be used. In one embodiment, preferably, the flavonoid is orally administered (fed).

The flavonoid represented by the formula (1) can be used to produce food, beverages, pharmaceutical products, quasi-pharmaceutical products, feed, or the like for use in promoting cartilage regeneration. In one embodiment, the present invention also encompasses use of the flavonoid represented by the formula (1) for producing the composition for promoting cartilage regeneration.

### EXAMPLES

The following describes the present invention in more detail with reference to examples, but the present invention is not limited to these examples.

### <Example 1>

Purchased human synovial cells (available from Toyobo Co., Ltd.; HFLS (Normal), cryopreserved cells (adult); Code No. CA40805a) were seeded into a 150 mm culture dish (available from Corning) at a low density of 50 cells/cm² and cultured for 14 days.

The human synovial cells were cultured in the following medium.
α-MEM (available from Nacalai Tesque Inc.)
10% Fetal bovine serum (available from Sigma-Aldrich) Antibiotic-Antimycotic Mixed Stock Solution (available from Nacalai Tesque Inc.)

Proliferated human synovial cells were detached with Accutase (available from Funakoshi Co., Ltd.), and the medium containing 20 × 10⁴ cells was placed in a 15 mL tube made of polypropylene, followed by centrifugation at 1000 × rpm for five minutes. The recovered human synovial cells were cultured in a medium containing a test compound shown in Table 1 for three weeks, and the weight of the resultant cartilage pellets was measured (N = 3, each). The medium containing the test compound was replaced once in two or three days. The test compound was dissolved in the medium using dimethyl sulfoxide (DMSO) (available from Nacalai Tesque Inc.; final concentration: 0.1%) to a final concentration of 50 µM. The test compound was taxifolin (available from Sigma-Aldrich) or hesperetin (available from Funakoshi Co., Ltd.).

The cartilage pellets were cultured in the following medium. The control was cultured by adding DMSO without test compounds to the medium described below (final concentration of DMSO: 0.1%).

### (Medium composition)

DMEM high-glucose (available from Thermo Fisher Scientific) 50 µg/mL L-ascorbic acid 2-phosphate sesquimagnesium salt hydrate (available from Sigma-Aldrich)
40 µg/mL L-proline (available from Sigma-Aldrich) 100 µg/mL Sodium pyruvate (available from Sigma-Aldrich) 50 mg/mL ITS + Premix (available from Corning)
10 ng/mL Transforming growth factor β3 (available from Sigma-Aldrich)
100 nM Dexamethasone (available from Sigma-Aldrich) 50 ng/mL Bone morphogenetic protein 2 (BMP-2) (available from Sigma-Aldrich)

Table 1 shows the evaluation results. In Table 1, the weight of pellets (g) is the weight of the cartilage pellets (average with N of 3), and SE is the standard error of the weight of pellets. Table 1 shows the rate of increase (%) for each test compound added, which is the value of the weight of the cartilage pellets relative to the weight of the cartilage pellets of the control taken as 100%. A significant difference in the rate of increase (%) determined from the weight of the cartilage pellets relative to the control was tested by Student's t-test, with the significance level being 5% or lower. Table 1 shows the results of the significant difference test. Taxifolin and hesperetin resulted in a significant increase in the weight of the cartilage pellets as compared to the control.

**[Table 1]**

| | Weight of pellets (g) (Ave) | SE | Rate of increase (%) | Significant difference |
|---|---|---|---|---|
| Control | 3.97 | 0.20 | - | - |
| Taxifolin | 5.73 | 0.23 | 144.5 | p < 0.05 |
| Hesperetin | 5.50 | 0.10 | 138.7 | p < 0.05 |

### <Example 2>

The test of Example 1 was repeated, except that quercetin (available from Nacalai Tesque Inc.), isorhamnetin (available from Funakoshi Co., Ltd.), or eriodictyol (available from Funakoshi Co., Ltd.) was used as a test compound, and the test compound in the medium was adjusted to a final concentration of 5 µM. Table 2 shows the weight of the cartilage pellets (g) (weight of pellets, average with N of 3) and the standard error (SE) of the weight of pellets. Table 2 shows the rate of increase (%) for each test compound added, which is the value of the weight of the cartilage pellets relative to the weight of the cartilage pellets of the control taken as 100%. A significant difference test was performed as in Example 1, with the significance level being 5% or lower.

**[Table 2]**

| | Weight of pellets (g) (Ave) | SE | Rate of increase (%) | Significant difference |
|---|---|---|---|---|
| Control | 3.63 | 0.09 | - | - |
| Eriodictyol | 4.80 | 0.20 | 132.1 | p < 0.05 |
| Isorham netin | 5.77 | 0.09 | 158.7 | p < 0.05 |
| Quercetin | 5.90 | 0.12 | 162.4 | p < 0.05 |

Quercetin, isorhamnetin, and eriodictyol resulted in an at least 130% increase in the weight of the cartilage pellets. Quercetin, isorhamnetin, and eriodictyol resulted in a significant increase in the weight of the cartilage pellets as compared to the control.

### <Example 3>

The test of Example 1 was repeated, except that luteolin (available from Funakoshi Co., Ltd.) or morin (available from Nacalai Tesque Inc.) was used as a test compound, and the test compound in the medium was adjusted to a final concentration of 5 µM. Table 3 shows the weight of the cartilage pellets (g) (weight of pellets, average of N of 3) and the standard error (SE) of the weight of pellets. Table 3 shows the rate of increase (%) for each test compound added, which is the value of the weight of the cartilage pellets relative to the weight of the cartilage pellets of the control taken as 100%. A significant difference test was performed as in Example 1, with the significance level being 5% or lower and the marginal significance level being 10% or lower. Morin resulted in a significant increase in the weight of the cartilage pellets as compared to the control. Luteolin also resulted in an increase in the weight of the cartilage pellets as compared to the control.

**[Table 3]**

| | Weight of pellets (g) (Ave) | SE | Rate of increase (%) | Significant difference |
|---|---|---|---|---|
| Control | 2.77 | 0.03 | - | - |
| Luteolin | 3.53 | 0.35 | 127.71 | p < 0.1 |
| Morin | 4.00 | 0.15 | 144.58 | p < 0.05 |

Synovial cells include mesenchymal stem cells, and the mesenchymal stem cells differentiate into cartilage cells. In Examples 1 to 3, adding the test compounds resulted in an increase in the weight of the cartilage pellets as compared to the control. This means that these test compounds promoted differentiation of mesenchymal stem cells into cartilage cells.

### <Comparative Example 1>

The test of Example 1 was repeated, except that the compounds shown in Table 4 were used as test compounds and tested with N = 1. Table 4 shows the rate of increase (%) for each test compound added, which is the value of the weight of the cartilage pellets relative to the weight of the cartilage pellets of the control taken as 100%. The final concentration of each of the compounds Nos. 1 to 15 in the medium was adjusted to 5 µM. The final concentration of each of the compounds Nos. 16 to 21 in the medium was adjusted to 50 µM.

**[Table 4]**

| No. | Compound | Rate of increase (%) |
|---|---|---|
| 1 | Astilbin | 60.5 |
| 2 | Apigenin | 104.7 |
| 3 | Cyanidin | 51.2 |
| 4 | Delphinidin | 41.9 |
| 5 | Myricetin | 69.8 |
| 6 | Daidzein | 100.0 |
| 7 | Genistein | 103.6 |
| 8 | Biochaninn A | 21.4 |
| 9 | Equol | 81.4 |
| 10 | 7,3',4'-Trihydroxyisoflavone | 95.3 |
| 11 | Calycosin | 95.3 |
| 12 | Formononetin | 100.0 |
| 13 | 3,4,2',4',6'-Pentahydroxychalcon | 110.7 |
| 14 | 4,2',4',6'-Tetrahydroxychalcon | 117.9 |
| 15 | Isoliquiritigenin | 82.1 |
| 16 | Epicatechin | 116.7 |
| 17 | Chrysin | 108.3 |
| 18 | Kaempferol | 95.8 |
| 19 | Naringenin | 116.7 |
| 20 | Catechin | 112.5 |
| 21 | Dihydrogenistein | 92.9 |

Each of the test compounds used in Examples 1 to 3 was the flavonoid represented by the formula (1), and the compound used in Comparative Example 1 was a polyphenol having a structure different from the formula (1). The test compounds used in Examples 1 to 3 each had a strong action that promotes differentiation of mesenchymal stem cells into cartilage cells, as compared to the compound of Comparative Example 1. The above shows that the flavonoid represented by the formula (1) has an excellent action that promotes differentiation into cartilage cells.

## Claims

1. A composition for promoting cartilage regeneration, comprising:
a flavonoid represented by the following formula (1) as an active ingredient: 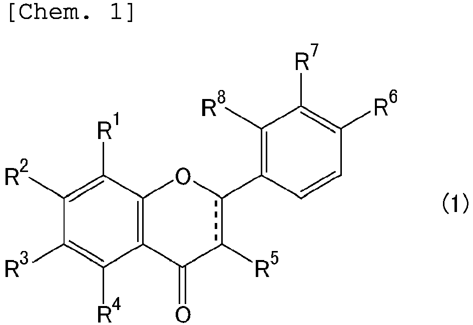 wherein R¹, R², R³, R⁴ and R⁵ each independently represent a hydrogen atom or a hydroxy group; R⁶ represents a hydroxy group or a C1-C2 alkoxy group; one of R⁷ and R⁸ represents a hydrogen atom and the other represents a hydroxy group or a C1-C2 alkoxy group; and a dashed line indicates that a bond may or may not be present.

2. The composition for promoting cartilage regeneration according to claim 1,
wherein the composition promotes differentiation into cartilage cells.

3. The composition for promoting cartilage regeneration according to claim 1 or 2,
wherein R¹ and R³ represent hydrogen atoms.

4. The composition for promoting cartilage regeneration according to any one of claims 1 to 3,
wherein R² and R⁴ represent hydroxy groups.

5. The composition for promoting cartilage regeneration according to any one of claims 1 to 4,
wherein the flavonoid represented by the formula (1) is a flavonoid represented by the following formula (2): 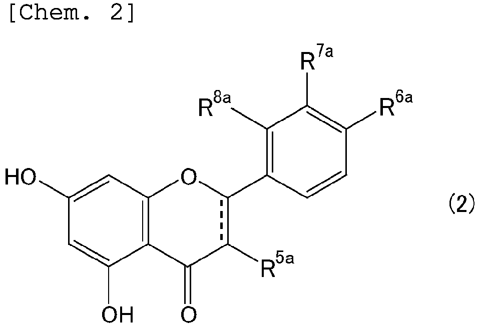 wherein R^{5a} represents a hydrogen atom or a hydroxy group; R^{6a} represents a hydroxy group or a methoxy group; one of R^{7a} and R^{8a} represents a hydrogen atom and the other represents a hydroxy group or a methoxy group; and a dashed line indicates that a bond may or may not be present.

6. The composition for promoting cartilage regeneration according to any one of claims 1 to 5,
wherein the flavonoid represented by the formula (1) is at least one selected from the group consisting of quercetin, hesperetin, taxifolin, isorhamnetin, eriodictyol, luteolin, and morin.

7. A method of promoting cartilage regeneration, comprising:
administering a flavonoid represented by the following formula (1): 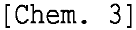 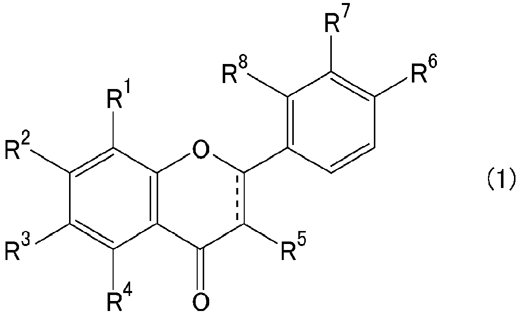 wherein R¹, R², R³, R⁴ and R⁵ each independently represent a hydrogen atom or a hydroxy group; R⁶ represents a hydroxy group or a C1-C2 alkoxy group; one of R⁷ and R⁸ represents a hydrogen atom and the other represents a hydroxy group or a C1-C2 alkoxy group; and a dashed line indicates that a bond may or may not be present.

8. Use of a flavonoid represented by the following formula (1) for promoting cartilage regeneration: 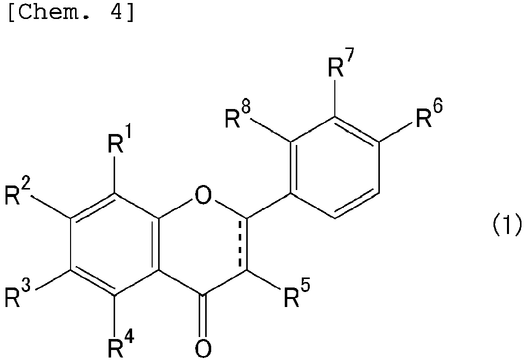 wherein R¹, R², R³, R⁴ and R⁵ each independently represent a hydrogen atom or a hydroxy group; R⁶ represents a hydroxy group or a C1-C2 alkoxy group; one of R⁷ and R⁸ represents a hydrogen atom and the other represents a hydroxy group or a C1-C2 alkoxy group; and a dashed line indicates that a bond may or may not be present.
